# EUROPÄISCHE PATENTSCHRIFT

(11) **EP 2 412 406 B1**
(45) Veröffentlichungstag und Bekanntmachung des Hinweises auf die Patenterteilung: **14.10.2015**
(21) Anmeldenummer: 11173520.5
(22) Anmeldetag: 12.07.2011
(51) Int. Cl.: A61N 7/00, A61B 19/00, A61N 7/02, A61B 17/00

(54) **Medizinischer Roboter und medinzinischer Arbeitsplatz**
Medical robot and medical work place
Robot médical et poste de travail médical

(30) Priorität: 26.07.2010 DE 102010038427
(43) Veröffentlichungstag der Anmeldung: 01.02.2012
(73) Patentinhaber: KUKA Laboratories GmbH, 86165 Augsburg (DE)
(72) Erfinder: Neff, Thomas, 80339 München (DE)
(74) Vertreter: Patentanwälte Funk & Böss GbR

(56) Entgegenhaltungen:
- EP-A1- 2 070 479
- US-A- 6 128 522
- US-A1- 2005 154 431
- US-B1- 6 425 865

## Beschreibung

Die Erfindung betrifft einen medizinischen Roboter und einen medizinischen Arbeitsplatz.

Roboter im Allgemeinen sind Handhabungsmaschinen, die zur selbsttätigen Handhabung von Objekten mit zweckdienlichen Werkzeugen ausgerüstet und in mehreren Bewegungsachsen insbesondere hinsichtlich Orientierung, Position und Arbeitsablauf programmierbar sind. Roboter weisen üblicherweise einen Roboterarm mit mehreren Gliedern und programmierbare Steuerungen (Steuervorrichtungen) auf, die während des Betriebs die Bewegungsabläufe des Roboterarms steuern bzw. regeln.

Beispielsweise bei der Tumorbehandlung kann sogenannter hochintensiver fokussierter Ultraschall (HIFU, englisch: "high intensity focused ultrasound") zum zumindest teilweisen Zerstören von Tumorgewebe eingesetzt werden. Es handelt sich dabei um eine Anwendung von Ultraschall, bei der durch eine gezielte Schallbündelkonzentrierung Gewebe erhitzt und zerstört wird.

Der Ultraschall wird von entsprechenden Schallerzeugungsvorrichtungen erzeugt, die im bestimmungsgemäßen Betrieb mit der Haut des zu behandelnden Lebewesens z.B. mithilfe einer Wasser- oder Gel-Vorlaufstrecke gekoppelt werden. Nutzt man Wasser als Vorlauf, dann sollte dieses möglichst vollständig entgast sein, da sonst Luftblasen den Schall ablenken und ungewollt streuen könnten.

Die US 2006/0293598 A1 offenbart, die Schallerzeugungsvorrichtung für eine HIFU Behandlung an einen Roboterarm zu befestigen, um während der Behandlung die Schallerzeugungsvorrichtung entsprechend einer Bewegung des zu behandelnden Lebewesens nachzuführen.

Die Druckschrift US 2005/0154431 A1 offenbart einen medizinischen Roboter, an dessen Roboterarm eine Schallerzeugungsvorrichtung befestigt ist, die vorgesehen ist, ein Lebewesen mit einem hochintensiven fokussierten Ultraschall zu beaufschlagen. Die Schallerzeugungsvorrichtung umfasst Sensoren, um eine haptische Rückkopplung der Kraft, mit der die Schallerzeugungsvorrichtung gegen das Lebewesen drückt, an einen Operateur zu erhalten. Es kann ein Lastsensor vorgesehen sein, welcher vorgesehen ist, dass die Schallerzeugsvorrichtung mit dem Lebewesen in Kontakt bleibt. Der Lastsensor kann Teil eines Roboterarm Krafterzeugungsmechanismus sein. Die Aufgabe der Erfindung ist es, einen medizinischen Roboters anzugeben, an dem eine Schallerzeugungsvorrichtung befestigt ist, bei dem eine Kopplung zwischen einem mittels der Schallerzeugungsvorrichtung zu behandelnden Lebewesen und der Schallerzeugungsvorrichtung erreicht wird.

Die Aufgabe der Erfindung wird gelöst durch einen medizinischen Roboter gemäss Anspruch 1.

Erfindungsgemäß ist es demnach vorgesehen, den medizinischen Roboter, an dessen Befestigungsvorrichtung die Schallerzeugungsvorrichtung befestigt ist, für eine verbesserte Ankopplung derselben an das mittels der Schallerzeugungsvorrichtung zu behandelnde Lebewesen kraftgeregelt zu betreiben. Dadurch kann erreicht werden, dass z.B. trotz einer Bewegung des Lebewesens beispielsweise bedingt durch seine Atmung, stets die Schallerzeugungsvorrichtung mit der vorgegebenen Kraft gegen die Oberfläche des Lebewesens, z.B. dessen Haut, drückt.

Um einerseits eine zumindest ausreichende Kopplung zwischen dem Lebewesen und der Schallerzeugungsvorrichtung zu erreichen und andererseits die Schallerzeugungsvorrichtung nicht zu stark gegen das Lebewesen zu drücken, liegt vorzugsweise die vorgegebene Kraft zwischen 10N und 50N.

Der Roboter kann eingerichtet sein, automatisch mit seinem Roboterarm die Schallerzeugungsvorrichtung an das Lebewesen heran zu bewegen.

Der Roboterarm kann manuell Bewegen, insbesondere handgeführt bewegt werden, um die an der Befestigungsvorrichtung befestigte Schallerzeugungsvorrichtung an die Oberfläche des Lebewesens heranzuführen. Während des manuellen Führens kann vorzugsweise gleichzeitig ein durch die Steuervorrichtung gesteuertes schwerkraftgeregeltes Betreiben der Antriebe erfolgen. Dadurch kann der Roboterarm relativ einfach und auch sicher manuell geführt werden.

Es kann vorgesehen sein, die Schallerzeugungsvorrichtung mittels des Roboters entlang einer vorgegebenen Bahn bei gleichzeitigem kraftgeregeltem Betreiben der Antriebe zu bewegen, sodass während der Bewegung der Schallerzeugungsvorrichtung entlang der Bahn der Roboterarm die Schallerzeugungsvorrichtung mit der vorgegebenen Kraft gegen das Lebewesen drückt. Der erfindungsgemäße medizinische Roboter ist insbesondere vorgesehen, mittels des von der Schallerzeugungsvorrichtung erzeugten Ultraschalls das Lebewesen z.B. im Rahmen einer Tumorbehandlung zu behandeln. Dazu sollte der Fokus des Ultraschalls möglichst im Tumor liegen. Außerdem kann es vorgesehen sein, den Tumor von verschiedenen Seiten mit dem Ultraschall zu beaufschlagen. Mittels Bilder vom Tumor, die vor der Behandlung z.B. mit einem medizintechnischen Gerät aufgenommen wurden, kann die Behandlung geplant werden, d.h. gegebenenfalls der Roboter derart programmiert werden, dass sich die Schallerzeugungsvorrichtung entsprechend der Planung bewegt. Da während der Bewegung der erfindungsgemäße Roboter gemäß dieser Variante kraftgeregelt betrieben wird, bleibt die relativ gute Kopplung zwischen der Schallerzeugungsvorrichtung und dem Lebewesen auch während der Bewegung entlang der Bahn erhalten. Insbesondere kann es vorgesehen sein, den Roboter in eine oder mehrere Raumrichtungen kraftgeregelt zu betreiben. In den Raumrichtungen, in denen der Roboter nicht kraftgeregelt wird, kann er z.B. positionsgeregelt betrieben werden, um beispielsweise eine Trajektorie oder Bahn abzufahren.

Gemäß einer Ausführungsform des erfindungsgemäßen Roboters ist an dessen Befestigungsvorrichtung zusätzlich ein bildgebendes medizintechnisches Gerät befestigt. Dann ist es möglich, wenigstens ein Bildes oder einen Bilddatensatze mittels eines an der Befestigungsvorrichtung befestigten bildgebenden medizintechnischen Gerätes während des Beaufschlagens des Lebewesens mit dem hochintensiven fokussierten Ultraschall zu erstellen, das Bild oder den Bilddatensatz auszuwerten, und abhängig von dem ausgewerteten Bild oder des ausgewerteten Bilddatensatzes, einen Fokus der Schallerzeugungsvorrichtung einzustellen oder den Roboterarm zu bewegen, sodass der Fokus der Schallerzeugungsvorrichtung auf ein zu Behandelndes Gewebe des Lebewesens fällt.

Aufgrund des bildgebenden medizintechnischen Gerätes, das insbesondere ein Ultraschallgerät ist, ist es demnach möglich, während der Behandlung mit der Schallerzeugungsvorrichtung ein Bild oder Bilder vom Lebewesen, insbesondere von dessen behandelndem Gewebe zu erstellen. Dieses Bild kann z.B. auf einem Sichtgerät angezeigt werden, sodass der Fokus der Schallerzeugungsvorrichtung gegebenenfalls manuell nachgestellt werden kann. Vorzugsweise wird jedoch das Bild bzw. der Bilddatensatz automatisch ausgewertet, um gegebenenfalls den Fokus der Schallerzeugungsvorrichtung automatisch nachzustellen bzw. diese mittels des Roboterarms zu bewegen.

Ein weiterer Aspekt der Erfindung betrifft einen medizinischer Arbeitsplatz, der im Wesentlichen mehrere erfindungsgemäßen medizinische Roboter umfasst, sodass das Lebewesen gleichzeitig mit wenigstens zwei Schallerzeugungsvorrichtungen behandelt werden kann, die jeweils von Roboterarmen kraftgeregelt gegen das Lebewesen gedrückt werden. Somit ist es möglich, die Zeitdauer der Behandlung des Lebewesens zu verkürzen.

Es ist insbesondere vorgesehen, das Lebewesen mittels Hochintensitäts-fokussiertem Ultraschall (Englisch: "highintensity focused ultrasound" - HIFU) zu behandeln. HIFU ist eine Möglichkeit, mit Ultraschallwellen gezielt Gewebe zu erhitzen und damit zu zerstören.

Es wird die Schallerzeugungsvorrichtung, insbesondere ein Ultraschall-Transducer verwendet, der es ermöglicht, den erzeugten Schallkegel so zu fokussieren, dass an einer gewünschten Stelle die Energie so hoch wird, dass das dort liegende Gewebe erhitzt wird. Die Erhitzung erfolgt vorzugsweise in einer relativ begrenzten fokalen Zone auf beispielsweise 60 bis 80 °C. Dies führt zu einer Gewebenekrose.

Um den Ultraschall möglichst gut in das Lebewesen einzuleiten, sollte zwischen der Schallerzeugungsvorrichtung, z.B. dem Transducer, und dem Lebewesen keine Luft vorhanden sein, da der Schall an der Luftgrenze zumindest teilweise, wenn nicht gar komplett reflektiert wird.

Je nach Ausführungsform stellt die Erfindung ein Robotersystem bereit, an dessen Flansch bzw. Befestigungsvorrichtung eine insbesondere als HIFU-Transducer ausgebildete Schallerzeugungsvorrichtung befestigt ist und die mittels Kraftregelung während der Behandlung vorzugsweise ständig den Transducer mit einer vorgegebenen Kraft auf die Hautoberfläche des Lebewesens drückt. Dieses System kann folgende Vorteile aufweisen:

Es ermöglicht eine extrakorporale HIFU-Behandlung ohne Wasserbecken, wodurch die Behandlung flexibler und auch nicht invasiv ausgeführt werden kann.

Es kann sich eine konformale Behandlung mit relativ frei wählbaren und kombinierbaren Einstrahlrichtungen ergeben, wodurch gegebenenfalls eine Anpassung der Dosis an den Tumor an das aktuelle Lebewesen bzw. an die aktuelle Behandlung verbessert werden kann.

Es wird eine adaptive Behandlung mit Kompensation von Tumorbewegungen durch Kraftsensorik und/oder online Bildgebung ermöglicht, wodurch eine Anpassung der Behandlung auf die aktuelle morphologische Situation ermöglicht wird.

Es kann die Möglichkeit einer Multi-Transducer-Behandlung durch Einsatz mehrerer Roboterarme vorgesehen sein, wodurch die Behandlung verkürzt werden kann.

Der erfindungsgemäße medizinische Roboter ist mit Kraftregelungseigenschaften ausgestattet. Die zu regelnde Kraft kann vorzugsweise parametrierbar sein.

Zusätzlich kann eine Schwerkraftregelung zur Handführung vorgesehen sein. Damit kann der erfindungsgemäße Roboter z.B. auf die ungefähre Zielposition durch manuelles Führen vorpositioniert werden kann.

Es wird ermöglicht, die Schallerzeugungsvorrichtung insbesondere in Form des Transducer während der Behandlung auf der Körperoberfläche des Lebewesens zu bewegen, ohne die Anpresskraft des Transducers zu verändern. Außerdem ist es möglich, dass während der Bewegung der Fokus des Ultraschalls immer im Tumor bleibt.

Durch den Einsatz des erfindungsgemäßen Roboters kann im Planungsschritt sehr viel flexibler geplant werden, da die Einstrahlrichtungen im Gegensatz zu konventionellen Systemen flexibler sind. Gegebenfalls Hindernisse zwischen dem zu behandelnden Gewebe und der Schallerzeugungsvorrichtung, wie beispielsweise Knochen oder Luft, können relativ leicht durch eine variierende Einstrahlrichtung umgangen werden.

Gegebenfalls durch ein Trackingsystem (Roboter und Lebewesen werden getrackt) kann der erfindungsgemäße Roboter die geplanten Zielpositionen relativ genau anfahren.

Es kann eine adaptive Behandlung realisiert werden. Der erfindungsgemäße Roboter kann in einem Komplettsystem als Teil einer Behandlungskette eingesetzt werden. Beispielsweise durch online Bildgebung mit einem bildgebenden medizintechnischen Gerät, insbesondere mit einem Ultraschall-Transducer, der ebenfalls an der Befestigungsvorrichtung befestigt sein kann und mit der Schallerzeugungsvorrichtung in einer fixen Relation steht, können Bewegungen des zu behandelnden Gewebes während der Behandlung erkannt und gegebenenfalls ausgeglichen werden. Auch Veränderungen zwischen dem Aufnahmezeitpunkt der Planungsbilder und der Behandlung können erkannt und gegebenenfalls ausgeglichen werden. Durch unterschiedlichen Füllungsgrad z.B. von Hohlorganen wie der Blase kann sich die Position des zu behandelnden Gewebes verändern. Außerdem können Atembewegungen auf der Hautoberfläche mittels Kraftsensorik erkannt und mit Hilfe der Kraftregelung kompensiert werden.

Gegebenfalls kann eine Multi-Transducer-Behandlung erfolgen. Durch gegebenenfalls Einsatz eines zweiten oder sogar dritten Roboterarms kann eine noch verbesserte Konvergenz erzielt werden, d.h. gegebenenfalls kann die Dosis durch Überlagerung mehrerer Foci verstärkt werden oder aber die Behandlung kann durch mehrere unabhängige Foci stark verkürzt werden.

Ausführungsbeispiele der Erfindung sind exemplarisch in den beigefügten schematischen Zeichnungen dargestellt. Es zeigen:
- Fig. 1: einen medizinischen Arbeitsplatz,
- Fig. 2: eine Vorrichtung zum Erzeugen von hochintensivem fokussiertem Ultraschall und
- Fig. 3: einen weiteren medizinischen Arbeitsplatz.

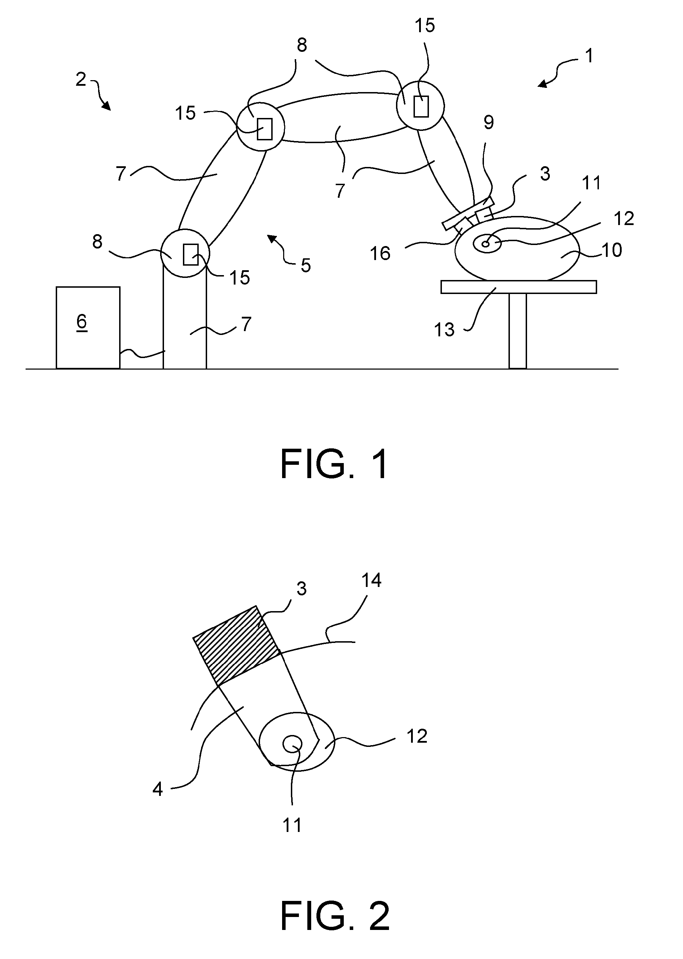

Die Fig. 1 zeigt einen medizinischen Arbeitsplatz 1 mit einem medizinischen Roboter 2, an dem eine Schallerzeugungsvorrichtung 3 befestigt ist, die eingerichtet ist, einen so genannten hochintensiven fokussierten Ultraschall zu erzeugen, dessen Schallkegel 4 in der Fig. 2 gezeigt ist.

Der Roboter 2, der insbesondere als ein Leichtbauroboter ausgeführt ist, weist im Falle des vorliegenden Ausführungsbeispiels einen Roboterarm 5 und eine die Bewegung des Roboterarms 5 steuernde Steuervorrichtung 6 auf. Der Roboterarm 5 weist mehrere Glieder 7 auf, die über Gelenke 8 verbunden sind. Die Glieder 7, die mittels Antriebe 15 bewegbar sind, können somit bezüglich Bewegungsachsen bewegt werden. Die Antriebe 15 sind insbesondere geregelte Antriebe, z.B. geregelte elektrische Antriebe, die von der Steuervorrichtung 6 angesteuert werden. Der Roboterarm 5 kann z.B. bezüglich sechs oder auch mehr Freiheitsgraden bewegt werden.

Der Roboterarm 5 weist eine Befestigungsvorrichtung 9 beispielsweise in Form eines Flansches auf. An der Befestigungsvorrichtung 9 ist die Schallerzeugungsvorrichtung 3 befestigt. Diese ist im Falle des vorliegenden Ausführungsbeispiels vorgesehen, dass mit dieser ein Lebewesen 10 behandelt wird. Das Lebewesen 10 liegt z.B. auf einer Patientenliege 13.

Insbesondere ist die Schallerzeugungsvorrichtung 3 vorgesehen, dass mittels des von ihr erzeugten hochintensiven fokussierten Ultraschalls ein Tumor 11 z.B. der Leber 12 des Lebewesens 10 behandelt wird. Damit der von der Schallerzeugungsvorrichtung 3 erzeugte hochintensive fokussierte Ultraschall den Tumor 11 erreichen und erhitzen kann, ist die Schallerzeugungsvorrichtung 3 während der Behandlung mit der Oberfläche des Lebewesens 10, insbesondere mit dessen Haut 14 gekoppelt.

Im Falle des vorliegenden Ausführungsbeispiels ist es vorgesehen, dass während der Behandlung des Lebewesens 10 der Roboter 2, insbesondere der Roboterarm 5 gesteuert durch die Steuervorrichtung 6 die Schallerzeugungsvorrichtung 3 automatisch mit einer vorab eingestellten Kraft gegen das Lebewesen 10 drückt, um zumindest eine zufriedenstellende Kopplung der Schallerzeugungsvorrichtung 3 mit der Haut 14 des Lebewesens 10 zu erhalten.

Damit der Roboterarm 5 mit der eingestellten Kraft die Schallerzeugungsvorrichtung 3 gegen das Lebewesen 10 drückt, läuft im Fall des vorliegenden Ausführungsbeispiels auf der Steuervorrichtung 6 ein Rechenprogramm, das den Roboterarm 5 bzw. die Antriebe 15 des Roboterarms 5 derart ansteuert, dass diese kraftgeregelt die Befestigungsvorrichtung 9 und somit die Schallerzeugungsvorrichtung 3 gegen das Lebewesen 10 mit der vorgegebenen Kraft drücken. Die vorgegebene Kraft ist beispielsweise 10N bis 50N. Somit ergibt sich zumindest eine zufriedenstellende Kopplung zwischen der Schallerzeugungsvorrichtung 3 und dem Lebewesen 10 während der Behandlung auch wenn sich das Lebewesen 10 beispielsweise aufgrund von Atmung relativ zur Schallerzeugungsvorrichtung 3 bewegt. Der Roboter 2 kann derart ausgeführt sein, dass er mittels seines Roboterarms 5 automatisch die Schallerzeugungsvorrichtung 3 an das Lebewesen 10 heranbewegt. Der Roboter 2 kann auch derart ausgeführt sein, dass er manuell an das Lebewesen 10 heranbewegt, insbesondere herangeführt wird. Dies kann z.B. dadurch realisiert werden, indem eine nicht näher dargestellte Person, beispielsweise ein das Lebewesen 10 behandelnder Arzt, dem Roboterarm 5 durch Ziehen und/oder Drücken an das Lebewesen 10 heranführt. Insbesondere in diesem Fall kann der Roboter 2 eine Schwerkraftregelung umfassen. Dies kann z.B. dadurch realisiert sein, indem auf der Steuervorrichtung 6 ein Rechenprogramm läuft, das während des manuellen Führens die Antriebe 15 derart ansteuert, sodass diese das manuelle Führen unterstützen.

Im Falle des vorliegenden Ausführungsbeispiels wird vor der Behandlung des Lebewesens 10 mittels eines bildgebenden, medizintechnischen Gerätes, z.B. mittels eines Ultraschallgerätes, eines Röntgengerätes, eines Computertomografiegerätes oder eines Magnetresonanzgerätes wenigstens ein Bild bzw. ein Bilddatensatz vom Tumor 11 erstellt und aufgrund dieses Bildes bzw. Bilddatensatzes die Behandlung geplant. Während der Behandlung kann es vorgesehen sein, dass der Tumor 11 aus verschiedenen Richtungen mit dem Ultraschall der Schallerzeugungsvorrichtung 3 beaufschlagt wird. Dazu kann der Roboter 2 eingerichtet sein, aufgrund der Planung automatisch den Roboterarm 5 zu bewegen. Während der Bewegung ist der Roboter 2 kraftgeregelt, sodass der Roboterarm 5 die Schallerzeugungsvorrichtung 3 mit der vorgegebenen Kraft gegen das Lebewesen 10 drückt. Es kann auch eine manuelle Bewegung während der Behandlung vorgesehen sein, wobei es vorgesehen sein kann, dass aufgrund der Kraftregelung der Roboterarm 5 die Schallerzeugungsvorrichtung 3 mit der vorgegebenen Kraft gegen das Lebewesen 10 drückt.

Im Falle des vorliegenden Ausführungsbeispiels kann es noch vorgesehen sein, dass zusätzlich zur Schallerzeugungsvorrichtung 3 ein bildgebendes medizintechnisches Gerät an der Befestigungsvorrichtung 9 des Roboterarms 5 befestigt ist. Dieses Gerät, das insbesondere ein Ultraschallgerät 16 ist, ist insbesondere in einer bekannten Relation zu Schallerzeugungsvorrichtung 3 an der Befestigungsvorrichtung 9 befestigt. Mittels des bildgebendes medizintechnisches Gerät bzw. des Ultraschallgerätes 16 kann z.B. während der Behandlung wenigstens ein Bild vom Tumor 11 erstellt werden, um beispielsweise automatisch den Roboterarm 5 derart nachzuführen, sodass ein Fokus des von der Schallerzeugungsvorrichtung 3 erzeugten Ultraschalls möglichst im Tumor 11 liegt. Es ist auch möglich, dass aufgrund des mittels des bildgebenden medizintechnischen Gerätes der Fokus der Schallerzeugungsvorrichtung 3 automatisch nachgestellt wird. Dazu sind z. B. die Schallerzeugungsvorrichtung 3 und das Ultraschallgerät 16 in nicht dargestellter Weise mit der Steuervorrichtung 6 verbunden. Es ist auch möglich, dass das mittels des bildgebenden medizintechnischen Gerätes erstellte Bild auf einem nicht näher dargestellten Sichtgerätes dargestellt wird, sodass gegebenenfalls beispielsweise der das Lebewesen 10 behandelnde Arzt den Fokus der Schallerzeugungsvorrichtung 3 nachstellen kann.

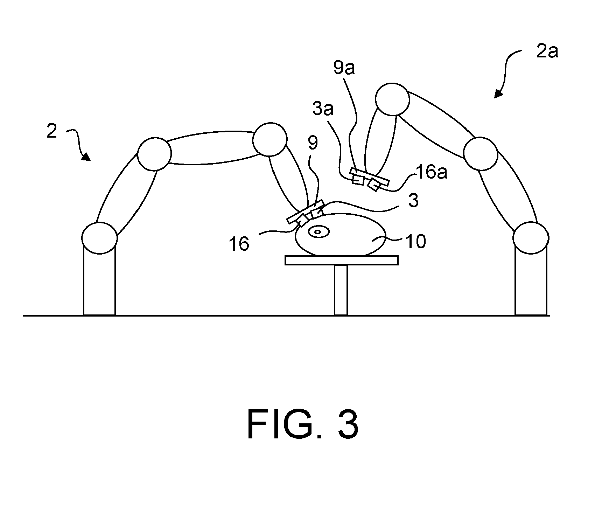

Die Fig. 3 zeigt einen weiteren medizinischen Arbeitsplatz, der zusätzlich zum Roboter 2 wenigstens einen weiteren Roboter 2a umfasst. An der Befestigungsvorrichtung 9a dessen Roboterarm ist ebenfalls eine Schallerzeugungsvorrichtung 3a befestigt, die eingerichtet ist, einen hochintensiven fokussierten Ultraschall zu erzeugen, um das Lebewesen 10 zu behandeln. Der Roboter 2a ist ebenfall kraftgeregelt, sodass dessen Schallerzeugungsvorrichtung 3a mit einer vorgegeben Kraft gegen das Lebewesen 10 drückt.

An der Befestigungsvorrichtung 9a des weiteren Roboters 2a kann ebenfalls ein bildgebendes medizintechnisches Gerät, z.B. ein Ultraschallgerät 16a befestigt werden. Die beiden Roboter 2, 2a können im Wesentlichen identisch ausgeführt sein. Mittels der beiden Roboter 2, 2a kann das Lebewesen 10 gleichzeitig behandelt werden.

## Patentansprüche

1. Medizinischer Roboter, aufweisend einen Roboterarm (5), der mehreren Glieder (7), zum Bewegen der Glieder (7) vorgesehene Antriebe (15) und eine Befestigungsvorrichtung (9) umfasst, eine an der Befestigungsvorrichtung (9) befestigte Schallerzeugungsvorrichtung (3), die vorgesehen ist, ein Lebewesen (10) mit einem hochintensiven fokussierten Ultraschall zu beaufschlagen, und eine Steuervorrichtung (6) zum Ansteuern der Antriebe (15), **dadurch gekennzeichnet, dass** die Steuervorrichtung (6) eingerichtet ist, die Antriebe (15) kraftgeregelt zu betreiben, sodass der Roboterarm (5) die Schallerzeugungsvorrichtung (3) während des Beaufschlagens des Lebewesens (3) mit dem hochintensiven fokussierten Ultraschall auf das Lebewesen (10) mit einer vorgegebenen Kraft drückt.

2. Medizinischer Roboter nach Anspruch 1, bei dem die vorgegeben Kraft zwischen 10N und 50N liegt.

3. Medizinischer Roboter nach Anspruch 1 oder 2, der eingerichtet ist, dass sein Roboterarm (5) manuell bewegbar, insbesondere führbar ist, um die an der Befestigungsvorrichtung (9) befestigte Schallerzeugungsvorrichtung (3) an die Oberfläche (14) des Lebewesens (10) heranzuführen, und dessen Steuervorrichtung (15) eingerichtet ist, gleichzeitig die Antriebe (15) schwerkraftgeregelt zu betreiben.

4. Medizinischer Roboter nach einem der Ansprüche 1 bis 3, der eingerichtet ist, die Schallerzeugungsvorrichtung (3) entlang einer vorgegebenen Bahn bei gleichzeitigem kraftgeregeltem Betreiben der Antriebe (15) zu bewegen, sodass während der Bewegung der Schallerzeugungsvorrichtung (3) entlang der Bahn der Roboterarm (5) die Schallerzeugungsvorrichtung (3) mit der vorgegebenen Kraft gegen das Lebewesen (10) drückt.

5. Medizinischer Roboter nach einem der Ansprüche 1 bis 4, zusätzlich aufweisend ein an der Befestigungsvorrichtung (9) befestigtes bildgebendes medizinisches Gerät (16), welches eingerichtet ist, wenigstens ein Bild oder einen Bilddatensatz während des Beaufschlagens des Lebewesens (10) mit dem hochintensiven fokussierten Ultraschall zu erstellen, und der Roboter (2) eingerichtet ist, aufgrund des ausgewerteten Bildes oder des Bilddatensatzes einen Fokus der Schallerzeugungsvorrichtung (3) einzustellen oder den Roboterarm (5) derart zu bewegen, sodass der Fokus der Schallerzeugungsvorrichtung (3) auf ein zu behandelndes Gewebe (11) des Lebewesens (10) fällt.

6. Medizinischer Arbeitsplatz, aufweisend den medizinischen Roboter nach einem der Ansprüche 1 bis 5 und einen weiteren medizinischen Roboter (6), der
- einen weiteren Roboterarm aufweist, der mehrere Glieder, zum Bewegen der Glieder vorgesehene weitere Antriebe und eine weitere Befestigungsvorrichtung (9a) umfasst,
- eine an der weiteren Befestigungsvorrichtung (9a) des befestigte weitere Schallerzeugungsvorrichtung (3a), die vorgesehen ist, das Lebewesen (10) miteinem hochintensiven fokussierten Ultraschall zu beaufschlagen, und
- eine weitere Steuervorrichtung zum Ansteuern der weiteren Antriebe, die eingerichtet ist, die weiteren Antriebe kraftgeregelt zu betreiben, sodass der weitere Roboterarm die weitere Schallerzeugungsvorrichtung (3a) während des Beaufschlagens des Lebewesens (10) mit dem Schall auf das Lebewesen (10) mit einer vorgegebenen Kraft drückt.

## Claims

1. Medical robot comprising a robot arm (5) which has a plurality of links (7), drives (15) provided for moving the links (7) and an attachment device (9), a sound generating device (3) secured to the attachment device (9), which sound generating device is provided for applying high intensity focused ultrasound to a living organism (10), and a control device (6) for activating the drives (15), **characterised in that** the control device (6) is set up to operate the drives (15) with force regulation, so that the robot arm (5) presses the sound generating device (3) against the living organism (10) at a specified force while the high intensity focussed ultrasound is being applied to the living organism (10).

2. Medical robot according to claim 1, in which the specified force is between 10N and 50N.

3. Medical robot according to either claim 1 or claim 2, which is configured so that its robot arm (5) can be moved manually, in particular guided, in order to move the sound generating device (3) secured to the attachment device (9) to the surface (14) of the living organism (10), and its control device (15) is set up at the same time to operate the drives (15) with gravitational force control.

4. Medical robot according to any one of claims 1 to 3, which is configured to move the sound generating device (3) along a predetermined path with the simultaneous force-regulated operation of the drives (15), so that during the movement of the sound generating device (3) along the path of the robot arm (5) the sound generating device (3) presses against the living organism (10) with the specified force.

5. Medical robot according to any one of claims 1 to 4, also comprising a medical imaging device (16) secured to the attachment device (9), which is configured to create at least one image or an image dataset during the application of the high intensity focussed ultrasound on the living organism (10), and the robot (2) is configured on the basis of the evaluated image or the image dataset to adjust a focus of the sound generating device (3) or to move the robot arm (5) such that the focus of the sound generating device falls on the tissue (11) of the living organism (10) to be treated.

6. Medical workstation comprising the medical robot according to any one of claims 1 to 5 and an additional medical robot (6) which
- comprises an additional robot arm which comprises a plurality of links, additional drives for moving the links and an additional attachment device (9a),
- an additional sound generating device (3a) secured to the additional attachment device (9a), which sound generating device is provided for applying a high intensity focused ultrasound to a living organism (10), and
- an additional control device for activating the additional drives, which is configured to operate the additional drives with force regulation, so that the additional robot arm presses the additional sound generating device (3a) against the living organism (10) with a specified force while sound is being applied to the living organism (10).

## Revendications

1. Robot médical présentant un bras de robot (5) qui comprend plusieurs membres (7), des entraînements (15) prévus pour mouvoir les membres (7) et un dispositif de fixation (9), un dispositif générateur de son (3) fixé sur le dispositif de fixation (9), qui est prévu pour solliciter un être vivant (10) avec un ultrason focalisé de haute intensité, et un dispositif de commande (6) pour piloter les entraînements (15), **caractérisé en ce que** le dispositif de commande (6) est configuré pour faire fonctionner les entraînements avec réglage de force, de telle sorte que le bras de robot (5) presse le dispositif générateur de son (3) sur l'être vivant (10) avec une force prédéterminée pendant la sollicitation de l'être vivant (10) avec l'ultrason focalisé de haute intensité.

2. Robot médical selon la revendication 1, dans lequel la force prédéterminée est comprise entre 10N et 50N.

3. Robot médical selon la revendication 1 ou 2, qui est configuré de telle sorte que son bras de robot (5) peut être déplacé, en particulier guidé manuellement pour rapprocher de la surface (14) de l'être vivant (10) le dispositif générateur de son (3) fixé sur le dispositif de fixation (9), et dont le dispositif de commande est configuré pour faire fonctionner en même temps les entraînements (15) réglés par la force de pesanteur.

4. Robot médical selon l'une des revendications 1 à 3, qui est configuré pour déplacer le dispositif générateur de son (3) le long d'une trajectoire prédéterminée en faisant fonctionner en même temps les entraînements (15) par la force de pesanteur, de telle sorte que pendant le déplacement du dispositif générateur de son (3) le long de la trajectoire, le bras de robot (5) presse le dispositif générateur de son (3) contre l'être vivant (10) avec la force prédéterminée.

5. Robot médical selon l'une des revendications 1 à 4, présentant additionnellement un appareil médical de visualisation (16) fixé sur le dispositif de fixation (9), qui est configuré pour créer au moins une image ou un jeu de données d'image pendant la sollicitation de l'être vivant (10) avec l'ultrason focalisé de haute intensité, et le robot (2) étant configuré pour ajuster un foyer sur la base de l'image évaluée ou du jeu de données d'image, ou pour déplacer le bras de robot (5) de telle sorte que le foyer du dispositif générateur de son (3) tombe sur un tissu (11) à traiter de l'être vivant (10).

6. Poste de travail médical présentant le robot médical selon l'une des revendications 1 à 5 et un autre robot médical (6) qui
- présente un autre bras de robot qui comprend plusieurs membres, d'autres entraînements prévus pour déplacer les membres et un autre dispositif de fixation (9a),
- un autre dispositif générateur de son (3a) fixé sur l'autre dispositif de fixation (9a), lequel est prévu pour solliciter l'être vivant (10) avec l'ultrason focalisé de haute intensité, et
- un autre dispositif de commande pour piloter les autres entraînements, qui est configuré pour faire fonctionner les autres entraînements par réglage de force, de telle sorte que l'autre bras de robot presse l'autre dispositif générateur de son (3a) sur l'être vivant (10) avec une force prédéterminée pendant la sollicitation de l'être vivant (10) avec le son.
